# EUROPEAN PATENT APPLICATION

(11) **EP 2 208 506 A1**
(43) Date of publication of application: **21.07.2010**
(21) Application number: 09075026.6
(22) Date of filing: 16.01.2009
(51) Int. Cl.: A61N 1/32, A61N 1/40

(54) **Intraluminar oncothermia catheter**

(71) Applicant: Oncotherm Kft., 2071 Páty (HU)
(72) Inventor: Szasz, Andras, 2071 Páty (HU); Szasz, Oliver, 2071 Páty (HU); Szasz, Nora, Boston, MA 02114 (US)
(74) Representative: Arth, Hans-Lothar

(57) **Abstract**

The present invention relates to a radiofrequency hyperthermia device for the treatment of intraluminar or intracavitary lesions consisting of a catheter with an electrode and RF-independent highly isolated temperature sensing, a counter-electrode and a radiofrequency source connected to the electrode and counter-electrode. The electrode which is fixed to the catheter is a good heat-conductor metallic electrode making direct galvanic- and heat-contact with the wall of the lumen/cavity and the material of the catheter has a high relative dielectric constant εᵣ and a low dielectric loss tgδ. The catheter is equipped by individual microchip, preferable mounted in its plug), which calibrates the catheter, guards its single use, collects the data and controls the proper use. The counter-electrode is positioned extracorporeal oppositely of the treated area so that the treated area is located between the electrode and the counter-electrode. The catheter is adjustable to different body lumens or body cavities such as urethra, rectum, esophagus, vagina, stomach, bladder, etc. and this radiofrequency hyperthermia device is useful for treatment of benign tumors or malignancies located within or close to these body lumens or body cavities by the use of radiofrequency (RF) fields in the range of 10 kHz to 45 MHz. The device could be remotely controlled by any web-browser installations independently its hardware solution (PDA, MDA, phone, PC, Mac, etc.)

## Description

The present invention relates to a radiofrequency hyperthermia device for the treatment of intraluminar or intracavitary lesions consisting of a catheter with an electrode and at least one means for fixing the position of the catheter in the body cavity, a counter-electrode and a radiofrequency source connected to the electrode and counter-electrode. The catheter is adjustable to different body lumens or body cavities such as urethra, rectum, esophagus, vagina, stomach, bladder, etc. and this radiofrequency hyperthermia device is useful for treatment of inflammation or malignancies located within or close to these body lumens or body cavities by the use of radiofrequency (RF) fields in the range of 10 kHz to 45 MHz.

Hyperthermia is widely used in medicine for the treatment of various diseases especially solid tumors. Still one basic problem of the hyperthermia treatment is the regioselectivity of the radiofrequency (RF) field especially for treatment areas located within or in the close neighborhood of body cavities or body lumens.

There are three general problems exist to construct the catheter applications in radiofrequency (RF) region of the frequency.
1. The non-homogeneous temperature distribution of the catheter treating area (generally a balloon). This could be solved by steered water circulation in the balloon. (EP 1 297 795 B1, Radiofrequency thermal balloon catheter).
2. The other great challenge is the unwanted heating of the area where the wire (RF cable) goes to the active treated volume. This is a principal problem of all the capacitively coupled systems, when the isolators (dielectric materials) are capacitive coupling media of the cable thought the path in the body. For example in the case of prostate treatment the heating of the penis is the real challenge. The shorter treatment time and the preferable air isolated cabling as well as circulation cold water could solve this problem.
3. The temperature measurement in the prostate is also a great challenge. Usually the temperature measured inside of the balloon of the treating catheter, far away from the really treated tissue and thermally isolated by the well isolating (in most of the cases silicon rubber, which is good thermal isolator) material of the catheter.
4. Fixing the catheters on the appropriate position is also a challenge to solve.

Thus objective of the present invention is to provide a radiofrequency hyperthermia device which can be used for the hyperthermia treatment of various body cavities and body lumens.

Our invention is to solve the challenges described above:
1. We have direct metallic or direct electrolyte contact with the lumen/cavity wall, without any thermal isolating (e.g. catheter rubber material). This makes the temperature distribution well homogeneous and controlled.
2. We have direct galvanic contact with the tissue, making higher electric conductivity by few orders of magnitudes in the treated connected surface than in the path of the RF-cable. This makes the RF-heating along the cable negligible, which could be well compensated with the blood-perfusion in the area.
3. We measure the temperature directly on a good thermal (and electrical) conductor, which allows a precise temperature measurement of the lumen/cavity wall. This is a safety issue (due to avoid the overheating of the lumen/cavity wall). On the other hand, due to the direct galvanic contact the RF-current is better controllable, and so the temperature distribution caused by the FR current is more homogeneous in the treated deep-situated tissue as well as the cancer treating electric field acts also better.
4. The catheter fixation is independent from the treatment electrode, i.e. from the RF electrode, so that the possible rearrangement caused by the treatment itself would not repositioning the catheter.

The usual locoregional hyperthermia by outside heatings, (capacitive, radiative, interference-focused, etc.) are not enough selective near the air-containing body cavities/lumens. The basic selection factors (tissue-density, tissue-impedance, tissue-heat-diffusion, blood-perfusion, etc.) are considerable modified by the cavities/lumens, the simple artificial or natural focusing mechanisms could be misleadingly affected. These complications demand the intracavital/intraluminar hyperthermia methods accompanying with the practically helpful advantage to be close to the treatable area.

The applied catheter/applicator has to be constructed to satisfy the following demands:
1. It is able to provide the modulated RF-signal, without heating the catheter itself. It is a problem with high frequency, because the wire in the dielectric material all along the catheter will be an antenna, heating up the path where the catheter is inserted.
2. It is able to measure the temperature accurately. The conventional tumor-therapies have a concept to apply the largest tolerable dose [mg/m²], [J/kg], measured in volume/mass dependent doses/values. Their efficacy is measured by off-situ diagnostics (e.g. MRI, CT, US, etc.). Their safety is measured by toxicity limit, established by dose-escalation studies and measured in the same doses as the therapy concept describes ([mg/m²], [J/kg]). The conventional temperature controlled hyperthermia measures everything in temperature-characterization: the concept is to apply the largest tolerable temperature [°C], the efficacy is measured by reached temperature in-situ [°C] and the safety limit is measured by hot-spots (temperature in-situ) [°C]. This last is a very important factor, because the only toxicity effect, which could cause the hyperthermia is the burning (hot spots). The temperature measurement in depth is necessary to avoid such (many times really dangerous) inside burnings. This makes the hyperthermia so complicated for quality guidelines, measured the temperature or invasively or they approximate it by complicated large devices like MRI. The temperature measurement is definitely a safety issue and not a treatment one. If the patient can not be heated as high as expected (large blood-flow like in liver or brain) or air cooling (breading) like in lung, or liquid-cooling (like in kidney or urinary tracks), that or the hyperthermia was not applied, or applied as it could be, without reaching any "temperature optimizing" issue. Opposite, if the temperature could be reached, but the patient tolerance limits the power, than the treatment again down regulated, the prescribed temperature is not reached again. Also the same limit is in action, when hot-spots arise outside the tumor, the energy intake must be limited to avoid the burn. In this case it is also problematic to reach the temperature guideline.
3. It is able to measure the temperature in average over a large area. The non-homogeneities has to be equalized and the temperature adjustments (control) has to be oriented on this average. No hot-spots allowed on the antenna surface.
4. It has to be safe, no burning could be caused to fix the catheter in the body. This requests a smooth and continuous touch with the internal cavity/lumen surface making the electric contact stable in all over the antenna surface. This means it has to be adjustable to the lumen/cavity-size (e.g. urethra, rectum, oesophagus, vagina, ... stomach, bladder, ... etc.). The poor, insecure connection could overload various areas by current and/or the high transition resistance could develop exceptionally high temperature in a spot. This is a danger not only during the treatment but afterwards it could block pulling out the applicator from the body (burned-in surface), which in extreme cases could be removed by open surgery only.
5. It must be resistant (together with its biocompatibility) against the chemically reactive body fluids/electrolytes, those must not modify the catheters during the treatment. Note the fluids are less aggressive in-vitro than in vivo; the joint oxidative and reductive reactions together with the destructive electrochemical effects during the treatment extremely load the treating applicator.
6. It has to be easily, safely and hygienically applicable, preferable on the same way, like the other routine catheters or endoscopic processes.
7. The applicator has to be identified and continuously controlled for documentation and traceability.
8. To fix the applicator in the desired position during the treatment is mandatory. (Note the treatment time is preferable 2-3 hours.)
9. The applicator must avoid multiple use. This has numerous reasons, like the hygienic problem, the resterilization could damage/change the material of the applicator, the catheter can not be identified if necessary for any after-treatment investigations. (Note this is a treatment applicator not a diagnostic one, so has more safety challenges than a normal endoscopy of catheterization.)

This objective is solved by the teachings of the independent claims. Further advantageous embodiments are evident from the dependent claims, the description, the examples and the figures.

The present invention relates to a radiofrequency hyperthermia device for the treatment of intraluminar or intracavitary lesions, preferable the tumorous (malignant or benign) lesions or other indications sensitive for heat/electric-current consisting of a catheter with an electrode and at least one means for fixing the position of the catheter in the body cavity, a counter-electrode and a radiofrequency source connected to the electrode and counter-electrode, wherein the electrode fixed to the catheter is a good heat-conductor metallic electrode and wherein the material of the catheter preferable has a relative dielectric constant εᵣ larger than 2 (εᵣ > 2) and preferable its dielectric loss tgδ is below 3•10⁻⁴ (tgδ < 3 • 10⁻⁴) and wherein the counter-electrode is positioned extracorporeal preferably oppositely of the treated area so that the treated area preferable is located between the electrode and the counter-electrode.

Especially the present invention relates to a radiofrequency hyperthermia device for the treatment of intraluminar or intracavitary lesions consisting of a catheter with an RF-electrode, wherein the RF-electrode has no thermal isolation and has a metallic part which is galvanically connected to the tissue and wherein said metallic part allows the precise temperature measurement and the homogeneous temperature distribution and an RF-independent temperature sensor which is isolated from the ground by at least 4kV, and a counter-electrode and a radiofrequency source connected to the RF-electrode and counter-electrode, wherein the RF-electrode fixed to the catheter is a heat-conductor metallic electrode and wherein the counter-electrode is positioned extracorporeal oppositely of the treated area so that the treated area is located between the electrode and the counter-electrode.

The RF-electrode of the catheter which is a heat-conductor metallic electrode has a heat-conductivity above 180 W/mK and preferably above 200 W/m/K.

In a preferred embodiment, the inventive radiofrequency hyperthermia device has a microchip controlled individual calibration and a data-collector. Also preferable is an embodiment of the radiofrequency hyperthermia device wherein a personalized treatment-procedure is programmed in and single-use guard and documentation. The catheter material is most preferably a biocompatible flexible high-temperature tubing, preferable silicon or silicolatex.

Concerning the radiofrequency source there is no limitation. Any common radiofrequency source can be used as long as the radiofrequency source is able to provide radiofrequency fields in the range of preferably 10 kHz to 50 MHz, and could be safely controlled according to the treatment demands. The range of frequencies supplied can actually be from below detectable the limit (effectively measured as 0 MHz) to 500 MHz, preferably from 10 kHz to 100 MHz, more preferably from 10 kHz to 45 MHz and most preferably 13.56 MHz or any value obtained by multiplication or division by an integer, preferable division by 40. Thus, the following frequencies are most preferred: 13.56 MHz, or 1/100, 1/40, 1/20, 1/10, ½ times, 2 times or 3 times, etc. this value (i.e. 6.78 MHz, 27.12 MHz or 40.68 MHz). The low frequencies are preferred to make definite conduction conditions instead of the radiation in the cavity/lumen. (The actually working device for prostate has 13.56/40 MHz = 339 kHz.)

Preferred is also that the frequency also called as carrier frequency is amplitude modulated in audio range.

The power supply to the electrodes can be symmetrical (coaxial) and continuous, or symmetrical (coaxial) and pulse supplied. Alternatively the power supply to the electrodes can be asymmetric (non-coaxial or partly coaxial) and continuous, or asymmetrical (non-coaxial or partly coaxial) and pulse supplied.

The treated body cavities or body lumens are all regions which can be reached by catheters such as urethra, rectum, esophagus, vagina, stomach, bladder, etc. and the catheter is adjusted in size to the body lumen or body cavity which should be treated.

Intraluminar or intracavitary **lesions** which can be treated by means of the inventive radiofrequency hyperthermia device are, for instance, cancer in prostate central/periphery area, benign prostate hyperplasia, prostatitis, esophagus cancer, bladder cancer, bladder cyst, stomach cancer, cervical carcinoma, vagina cancer, vagina cyst, cancer or papilloma-virus infected cervix-uteri, colon/rectal carcinomas, anus-carcinoma, etc..

As used herein, the term "oncothermia" refers to the treatment of intraluminar or intracavitary lesions in living bodies (humans and animals) by heat and electric field combination, induced by the radiofrequency current flow through the treated volume.

The term "hyperthermia" as used herein refers to heating up a target higher than the environment equilibrium (homeostatic) temperature.

The **catheter** used as component of the radiofrequency hyperthermia device is resistant against aggressive body fluids and electrolytes, so that such fluids or electrolytes do not modify the catheter (and its built-in electronics) during the treatment and do not alter the properties of the catheter during the treatment. Common catheters such as Foley-catheters can be used as long as an electrode can be positioned on the end of such a catheter. The electrode and the temperature sensors as well as the RF-filters are built in the Foley catheter/applicator and conductively connected to the radiofrequency source and to the controlling device.

The catheter or the radiofrequency device preferably further comprises at least one means for fixing the position of the catheter in the body cavity or body lumen, wherein this at least one means is not part of the RF electrode itself. Thus the catheter and especially the treatment electrode is fixed in its position even if possible rearrangements of the treated tissue occurs during treatment.
Preferably, the full catheter is microchip controlled (built in processor in its plug) for control the single use, for calibrate individually and for register and document for later statistical and/or legal use.

Moreover the catheter is able to provide the RF signal, preferably the modulated RF signal, without heating the catheter itself. This is very important to avoid burning of the tissue which is in direct contact to the catheter surface along the path to the treatable area.

Therefore it is important that the catheter and especially the catheter material which comes into contact with the tissue has a high relative dielectric constant and a low dielectric loss in the frequency range 5 - 500 MHz (ω = 3 • 10⁷ - 3 • 10⁹) and more preferred in the frequency range of 5 - 50 MHz (w = 3 • 10⁷ - 3 • 10⁸). Thus, the catheter material (of course except the electrode) is similar to an isolator and consists of a material which could also be used for isolators. As used herein, the term "high relative dielectric constant" refers to a relative dielectric constant (εᵣ) larger than 1.0 (εᵣ > 1.0), more preferably larger than 1.5 (εᵣ > 1.5) and most preferably larger than 2.0 (εᵣ > 2.0). As used herein, the term "low dielectric loss" refers to a dielectric loss (tgδ) lower than 11·10⁻⁴ (tgδ < 11 • 10⁻⁴), more preferably lower than 6·10⁻⁴ (tgδ < 6 • 10⁻⁴) and most preferably below than 3·10⁻⁴ (tgδ < 3 • 10⁻⁴)_{.}

Preferred materials or isolator materials for the catheter or at least for the part of the catheter which comes into contact with the tissue are for instance:

| | | |
|---|---|---|
| Teflon: | εᵣ=2.1, | tgδ=2·10⁻⁴; |
| Polyethylene: | εᵣ=2.3, | tgδ=2·10⁻⁴; |
| Silicon oil: | εᵣ=2.3, | tgδ=2·10⁻⁴; |
| Aluminium oxide (Al₂O₃): | εᵣ=8.6, | tgδ=10·10⁻⁴, |
| Quartz (SiO₂, fused): | εᵣ=3.8, | tgδ=1.5·10⁻⁴. |

One most preferred material for the catheter on the frequencies lower than 1 MHz is the Silcolatex material (Ruesch product).

The **electrode** or RF electrode or treatment electrode at the end of the catheter is in the form of a metal cylinder preferably having the same or identical diameter as the catheter tube. The RF electrode is located at the end of the catheter tube between the guiding head of the catheter tube and the balloon or below the guiding head and below the balloon or within the balloon.

The RF electrode is made of a good heat-conductor metallic cylinder. Its material is preferably selected from the group comprising copper, silver, gold, aluminium or iron, or their alloyed, coated, powder-manufactured or cermet forms. Most preferred is pure copper as material for the RF electrode.

In a more preferred embodiment the RF electrode is coated with gold or silver or a gold-silver alloy so that such coated aluminium or copper electrodes are more preferred. Most preferred is a RF electrode coated by a gold-silver alloy.

It was found that the gold is useful to avoid the sticky burning glue of the electrode which could block of pulling out the catheter after finishing the treatment. The silver is applied for better conduction, larger wear-resistance and antibacterial effect. The coating must have enough thickness to be continuous during the full treatment procedure, and the pull-out. Preferable a few microns (about 5) of gold-silver (50-50%) is satisfactory. The adhesion of the coating must be perfect to keep the layer not pilled out. The coating layer must cover perfectly (no leakages, no porosity) the base material due to the request of biocompatibility, except if the cylinder is biocompatible material like for example silver or gold. (Naturally, in the last cases no coating is necessary.)

In a preferred embodiment, the **catheter** contains or comprises means for temperature measurement. Said means for temperature measurement could be thermometers which are able to accurately measure the temperature preferably measure the temperature in average over a large area which is at least the treated area. The temperature measurement has to be "RF-tolerable" in a wide range of frequency (up to 45 MHz), so its measured value must not be modified by the active field or its fluctuations (derivatives). The means for temperature measurement could be a thermo-pair or thermistor or platinum-resistor or other temperature sensing structure / element. Preferable are micro-thermistors.

The measured temperature is preferably filtered with high level filters built in the catheter. Their role is to avoid incorrect thermo-signals caused by RF-field or by its induced currents, which could corrupt the measured signal. This filter is preferable a resonant serial circuit, (or in a simple case could be a condenser only, having low admittance in the given frequency) tuned to the applied frequency. Its resonance/conduction short circuits the actual RF, but does not modify the measuring signal of the thermo-element. This solution keeps the thermo-signal correct and intact from the RF-effects. The filter is preferable built in directly at every sensors (preferable smallest SMD parts 0.2 mm sizes) and preferable in the end-plug of the catheter to block the further induction modification.

Moreover it is preferred that the inventive radiofrequency hyperthermia device comprises a temperature sensing electric system. The temperature sensing electric system is preferably highly isolated (at least 4kV) for safety reasons. The thermistor resistivity is not measured by DC, which could be sensitive for RF-noises. The thermo-signal is measured with low frequency RF, far from the treatment frequency, (preferable 80 kHz at 339 kHz carrier and in audio-range modulation range) but giving enough signal for the preferable isolating transformer or opto-connection transmission to isolate on 4 kV standard value.

The wires or cables as well as all the electronic parts, which connect the intracorporeal electrode with the RF source, including the non-inert part of the thermo-sensors, is completely isolated from the body or body fluids by the use of a non-conductive material such as plastics or silicon and preferably flexible biocompatible elastosil N2010 (commercially available from manufacturer: Wacker, Germany, www.wacker.com).

It is preferred that the temperature is measured in the far ends of the good heat-conductor cylinder (the RF electrode), measuring the average temperature of the treated surface. The applied thermo-sensor has to be as near as or as close as possible to the surface and to the treated tissue. For the perfect thermal sensing the sensors are in direct tight contact with the RF-antenna-cylinder, preferable run through the catheter tube to the outside of the tube touching the inside of the cylinder.

In order to enable the cables to follow the elastic pull-of the catheter the cables are arranged in a spring-like loop within the catheter tube so that the length of the cables is not fixed and can be variable within a certain range provided by the diameter of the loop and the number of loops.

Moreover it is preferred that the thermo-wiring is a twisted-pair and is electronically protected (shielding, filtering, time-sharing, etc.).
In a preferred embodiment of the present invention the full cable-branch can be removed in case of emergency with a definite direct pull. In another more preferred embodiment said definite direct pull is connected with the immediate deflation of the catheter balloon in order to remove the complete catheter immediately.

Moreover it is preferred that the inventive radiofrequency device contains or comprises at least one device for treatment control with preprogrammed protocols, web-browser facility for remote control of the treatment by any web-access (laptop, PDA, MDA, phone, PC, Mac, etc.).

Essential to the present invention is the size and form and especially the relative position of the electrode and counter-electrode. The counter-electrode is positioned extracorporeal directly to the patient's skin and can be any commonly used electrode for hyperthermia applications using radiofrequency fields in the range of 10 kHz to 45 MHz.

Since the **counter-electrode** is not located within the catheter close to the electrode, this arrangement ensures that the RF-electrode itself and the catheter end itself is not warmed up or heated by the radiofrequency field. This is why the RF-electrode uses conduction heating and not radiation of RF or microwave. Thus this arrangement with an intracorporeal electrode and extracorporeal counter-electrode is able to regioselectively heat the diseases area of patients body by conductive (galvanically connected) method.

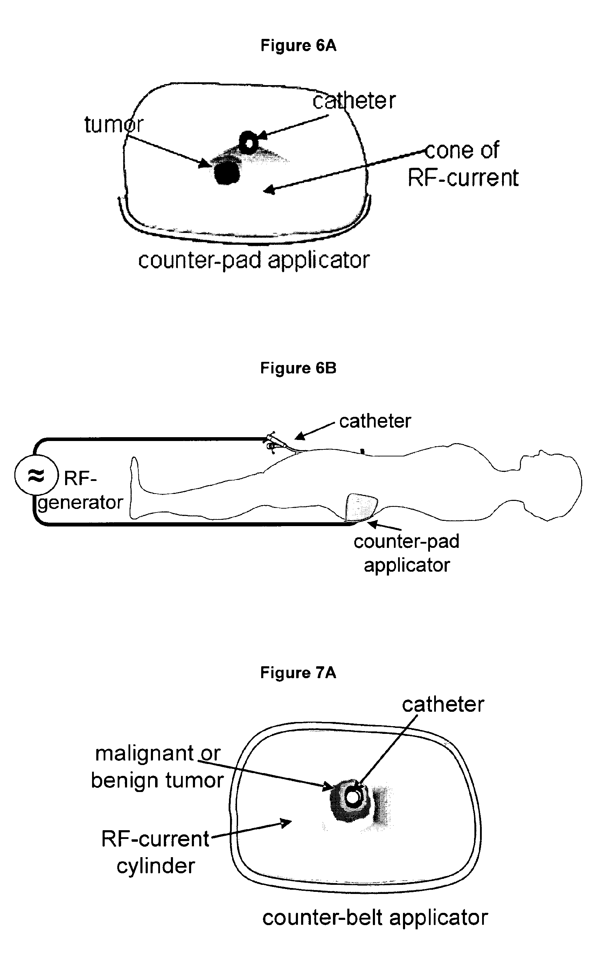

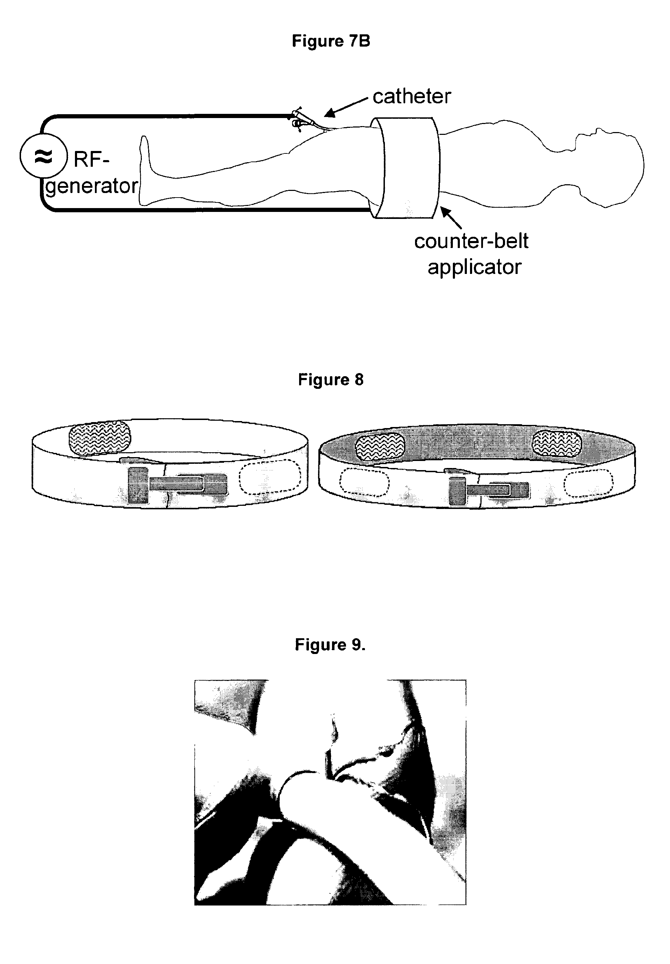

Figure 6 shows the arrangement of the inventive radiofrequency hyperthermia device with the RF source, the catheter inserted through the penis into the prostate and the counter-electrode positioned extracorporeal like a half belt around patient's back. This arrangement is useful for the treatment of prostate cancer. A similar arrangement is shown in Figure 7. The electrode is positioned in the prostate while the counter-electrode in Figure 6 is positioned directly on the patient's skin dorsal around patient's back from one lateral side to the other lateral side like a half belt.

In Figure 7 it is shown that the counter-electrode positioned on patient's skin goes fully around the patient like a belt referred to herein as full belt.

Since the area treated by radiofrequency hyperthermia is the area between the electrode and counter-electrode, the diseased area can regioselectively be treated by selecting a suitable position and size of the counter-electrode in relation to the intracorporeal electrode. The left picture of Fig. 6 shows the treated area as a triangle between the intracorporeal electrode and the half belt counter-electrode. The left picture of Figure 7 shows that the treated area is the whole area between the intracorporeal electrode and the outer counter-electrode in form of a full belt electrode which goes completely around the intracorporeal electrode.

Thus according to the present invention, the counter-electrode is positioned oppositely of the treated area in order to force the RF current flow through the desired region.

The counter-electrode is preferably in the form and shape of the belt or part of a belt (half belt, etc.) or the at least one counter-electrode is incorporated into a belt or bandage which can easily fixed preferably tightly fixed to the patient's body at a similar height where the intracorporeal electrode is located. The counter-electrode which means the at least one counter-electrode incorporated into a bandage or medical belt is also called herein belt electrode or bandage electrode.

The belt electrode or bandage electrode is flexible, expandable, elastic and/or stretchable, can be provided in any desired size and can be easily used and fixed to patient's body. The complete belt electrode or bandage electrode can form the conductive part of the counter-electrode or only parts of the belt electrode or bandage electrode can form the conductive area of the counter-electrode. These conductive parts or conductive areas can be made in any desired arrangement, shape, number and size. Preferably, also the conductive parts or conductive areas of the belt electrode or bandage electrode are flexible, expandable, elastic and/or stretchable so that they can adhere tightly to the area of the body which should be treated.

Figure 8 shows a belt electrode or belt-like shape electrode. The areas within the dotted lines and the areas with wavy lines indicate the conductive part of the electrode while the left belt electrode has two and the right belt electrode has four conductive parts or electrodes. However these are only two examples of such counter-electrodes. In other preferred examples the belt electrode or bandage electrode has the conductive part only dorsal (back-belt) or only lateral (side-belt) or only ventral (front-belt) or dorsal and lateral (e.g. half belt) or dorsal and ventral or ventral and lateral (e.g. half belt) or dorsal and ventral and lateral or completely around the patent's body (full belt).

In further preferred embodiments of the present invention the counter-electrode comprises a flexible preferably porous material, carrier or support coated with conductive metal.

The carrier or support or material and especially the solid carrier or solid support or solid material used for manufacturing the counter-electrode is made of plastic, polymers or natural substances such as biopolymers is coated with a conductive material such as a conductive metal or metal alloy. Moreover said coated carrier or coated support or coated material is porous and allows a liquid to pass through said carrier or support or material. Furthermore, the coated carrier or coated support or coated material is flexible, i.e. does not have a definite or predefined shape and is able to follow the uneven curvatures of the human or animal body.

Instead of a coated flexible, elastic or stretchable carrier or a coated flexible material a conductive metallic net or a conductive metallic network can be used manufactured of at least one conductive metal electrode material. Such metallic nets or metallic networks do preferably not comprise any backbone such as a polymeric network structure. The metallic net or network is preferably a woven structure of metallic fibres having very similar properties as the coated flexible material such a coated textile. The conductive metallic net or network is flexible, elastic or stretchable, allows water and other fluids as well as gases to move through it, can be folded without negative effect concerning conductivity and is able to cover uneven, fractal and/or percolative surfaces. Consequently all kind of metallic nets and networks having the afore-mentioned properties of the conductively coated materials such as the conductively coated textiles are useful for manufacturing the counter-electrode.

The term "porous" as used herein refers to the ability that the coated carrier or coated support or coated material allows water and any gas to move through said coated carrier or coated support or coated material. The pore size can be up to 0.1 mm or even larger.

A porous textile is suitable as the flexible material or flexible, elastic or stretchable carrier or flexible, elastic or stretchable support however any porous material having flexibility similar to the flexibility of a woven or non-woven textile could also be used in the present invention. Thus any kind of textile, woven textile, non-woven textile and even non-textile material is suitable as flexible porous material. Such a flexible porous material can also be named as a flexible porous solid support or flexible porous solid carrier. Such materials, carrier or supports are not limited by a specific shape and have the consistency and/or texture of piece of textile or a piece of fabric or drapery. Consequently all known natural and artificial materials such as polyamide (Nylon^{®}), poly-ε-caprolactone, poly-para-dioxanones, polyanhydrides, polyhydroxymethacrylates, fibrin, polyetherester, PEG, poly(butylene terephthalates), polycarbonates, poly(N-vinyl)-pyrrolidone, polyvinylalcohols, polyesteramides, polyethyleneoxide, polypropyleneoxide, polyurethanes, fibrinogen, starch, collagen, zein, casein, β-cyclodextrins, polyacrylates, polyacrylamide, polyimides, polyethylene, polypropylene, polytetrafluoroethylene, fluorosilicones, rayon, polysulphones, silicones, polysiloxanes, polyvinyl halogens and copolymers or mixtures of these substances.

Preferred are materials, carrier or supports such as these mentioned before which provide a good adhesion for the metallic coating. Also preferred are materials, carrier or supports which are manufactured of or which consist of a plurality of single fibres like a woven textile wherein one set of the single fibres extends along the complete length of the textile more or less in a substantially parallel manner while the other set of fibres is arranged in a substantially parallel manner diagonal to the first set of fibres. Thus fibres having a length which is similar to the length of the textile comprising said fibres are preferred.

In a further preferred embodiment of the present invention the single fibres of the material, carrier or support are coated like a tube that means not only a part of the surface of the fibre is coated rather the coating is applied all around the fibre.

Moreover it is preferred that such material, carrier or support is percolative and/or fractal or has a percolative and/or fractal structure without any discontinuity between the in and out cables of the RF source means. In other words, the metallic nets or the coated carriers are such flexible that they are able to cover uneven, fractal or percolative surfaces or they are able to follow the structure of uneven, fractal or percolative surfaces.

The conductive metal coating is a multilayer coating. Preferably one of the layers is silver which has a good antibacterial effect and provides for good radiofrequency (RF) conduction. Moreover silver has an anti-odour effect together with moderate anti-perspiration activity. This makes silver preferred for cosmetic, medical and well-being applications.

The textile can be coated by extra plastic layer for galvanic isolation. This should preferably not be a continuous layer, but a layer on the fibres only in order to keep the porous structure free and open. If the device is double isolated, direct metallic contact could be used. The multilayer structure coats the fibres co-axially and perfectly continuous. If the metal layer-making technology is dipping electroless process than the crossing of individual fibres could be also perfectly coated individually fibre by fibre, if it is galvanic, than the crossings could be coated only as a cross, not keeping the individual fibre co-axial structure. The plastic coating however has to be dipping with such surface tension of the bath, which does not allow the isolation of the metallic coated fibres at their crossing, only coats their outer surface, however the coating does not fills up the pores so the material remains porous.

Ideally the counter-electrodes are suitable for application to all parts of the human and animal body. For this reason there is a need for a flexible counter-electrode that can conform to the contours of the body. The coated flexible carrier or the flexible metallic net acts as an counter-electrode for the treatment of a large and/or uneven, fractal and/or percolative surface while these carriers or metallic nets can easily be fixed on said surface via a belt or bandage, or the like.

The preferred inventive flexible counter-electrode of the present invention is able to fulfill this requirement, i.e. to be conductive, flexible, foldable, porous and able to cover uneven, fractal and/or percolative surfaces smoothly like a cloth following the contour of the surface. The flexible counter-electrode is formed from a conductive metal coated flexible material or a metallic network that can be folded or formed freely even into cylinder and can be placed around patient's body. Thus the inventive electrode can conform to gradual and sharp curvatures. For example, in the case of sharp curvatures it can easily conform to the shape of a abdomen by wrapping around the abdomen. Alternatively, in the case of gradual curvatures it can conform to the shape of the torso.

Also the inventive flexible electromagnetically coupled counter-electrode is lightweight and therefore can be produced to cover a large area, such as the torso, without causing patient discomfort due to excessive weight of the counter-electrode. This allows the treatment of large areas in a single treatment session. Also the flexibility of the energy transfer means allows for good contact between the counter-electrode and a large application area, for example, the torso.

The inventive flexible electromagnetically coupled counter-electrode is also porous. This allows for natural cooling of the treatment area due to exchange of heat through the energy transfer means via convection. Also a simple external air cooling system, for example a directed air flow from a fan, can be used to cool the application area to prevent burning and maintain patient comfort. As a result there is no requirement for a complicated fluid cooling system as is the case in conventional bolus electrodes. This allows for a simple and lightweight construction that is suitable for home use by a patient

The porosity of the counter-electrode also allows for the exchange of fluids through the counter-electrode. Thus perspiration can evaporate naturally through the porous counter-electrode and therefore increase patient comfort.

Due to the simple construction of the preferred inventive flexible counter-electrode it can be provided as a single use disposable electrode or could be produced to specification for an individual patient. A further advantage of the inventive flexible counter-electrode is that there is no requirement for a bulky, rigid-frame and difficult to operate bolus electrode applicator.

The radiofrequency hyperthermia device of the present invention can be used to treat intraluminar or intracavitary lesions, cancer, tumors and malignancies. The intraluminar or intracavitary area in need of treatment could be an area within the urethra, prostate, penis, rectum, esophagus, vagina, uterus-cervix, anus, rectum, colon, sigma, stomach or bladder. Consequently the inventive radiofrequency hyperthermia device is useful for the prophylaxis, especially after-treatment or prophylaxis after a successful cancer treatment in order to prevent development of new cancer or new tumors and treatment of cancer, benign and malignant tumors, especially urethral lesions, malignant or benign prostate cancer, benign prostate hyperplasia, rectum lesions and rectum cyst, rectum cancer, colon cyst, colon cancer, anus cancer, anus cyst, esophagus lesions and esophagus cancer, cervical carcinoma, vagina cyst, vagina tumors, penis cancer, stomach cyst, stomach cancer or bladder cyst and bladder cancer.

The term "intraluminar or intracavitary lesions" as used herein also refers to cancer, tumors and malignancies or malignant diseases of gastroenterological, gynecological and andrological cavities and lumens.

Moreover the present invention is directed to a catheter for a radiofrequency hyperthermia device which is useful for the treatment of intraluminar or intracavitary lesions as defined herein consisting of an RF-electrode, wherein the RF-electrode has no thermal isolation against the contacted tissue and has a metallic part which is galvanically connected directly or by conductive electrolyte indirectly to the tissue and wherein said metallic part allows the precise temperature measurement and the homogeneous temperature distribution and an RF-independent temperature sensor which is isolated from the ground by at least 4kV, wherein the RF-electrode fixed to the catheter is a heat-conductor metallic electrode. The metallic electrode is coated with antibacterial and anti-stickiness coating avoids the infection and the sticky burn-fixed electrode after the treatment, which could blocks its pulling out from the lumen. The size of the metallic electrode fits to the lumen diameter, and fits in its lengths to the area of treatments. Especially in prostate it is Ch16 in lumen-size (5.3 mm diameter) and 1.8 cm length (the prostate interior, not risking the incontinence by heating of the closing muscle.

The RF-electrode has no thermal isolation against the contacted tissue means that the RF-electrode has a metallic contact (or direct metallic contact) or electrolyte contact with the surrounding lumen wall or cavity wall without any thermal isolation, for instance, through a rubber material on the catheter or electrode surface. This guarantees a homogeneous and controlled temperature distribution. The direct galvanic contact to the tissue, i.e. the missing thermal isolation is essential to the present invention, because that generates higher electric conductivity by few orders of magnitudes in the treated tissue than in the path of the RF-cable so that the diseased tissue is substantially heated and not the tissue along the way where the catheter was inserted into the body. Moreover the
the direct galvanic contact makes the RF-current better controllable so that the temperature distribution caused by the FR current is more homogeneous in the treated deep-situated diseased tissue which increases the treatment efficiency.

The afore-mentioned catheter can be used for the manufacture of a radiofrequency hyperthermia device for the treatment of intraluminar or intracavitary lesions consisting of said catheter with an RF-independent temperature sensor which is isolated from the ground by at least 4kV, wherein the RF-electrode fixed to the catheter is a heat-conductor metallic electrode, and a counter-electrode and a radiofrequency source connected to the RF-electrode and counter-electrode, wherein the RF-electrode fixed to the catheter is a heat-conductor metallic electrode and wherein the counter-electrode is positioned extracorporeal oppositely of the treated area so that the treated area is located between the electrode and the counter-electrode.

Consequently the present application relates also to a method for treating a patient including a human in need thereof with radiofrequency hyperthermia comprising inserting a catheter with an electrode connected to a RF source into a body lumen or body cavity and positioning the counter-electrode connected to said RF source so on the patient's skin that the area which should be treated is located between the electrode and the counter-electrode and applying a radiofrequency field in the rang of 10 kHz to 45 MHz in order to heat up the diseased area between the electrode and counter-electrode.

Since the present hyperthermia or oncothermia treatment does not have any noteworthy side effects, the hyperthermia or oncothermia treatment can perfectly be used in combination with common chemotherapy treatment. Thus, the inventive radiofrequency hyperthermia device can perfectly be used in combination with chemotherapy treatment with cytostatic, anti-proliferative and/or cytotoxic drugs. Example of some cytostatic, anti-proliferative and/or cytotoxic drugs are actinomycin D, aminoglutethimide, amsacrin, anastrozol, antagonists of purine and pyrimidine bases, anthracycline, aromatase inhibitors, asparaginase, antiestrogenes, bexaroten, bleomycin, buselerin, busulfan, camptothecin derivates, capecitabin, carboplatin, carmustine, chlorambucil, cisplatin, cladribin, cyclophosphamide, cytarabin, cytosinarabinoside, alkylating cytostatics, dacarbacin, dactinomycin, daunorubicin, docetaxel, doxorubicin (adriamycin), doxorubicin lipo, epirubicin, estramustine, etoposid, exemestan, fludarabin, fluorouracil, folic acid antagonists, formestan, gemcitabin, glucocorticoides, goselerin, hormones and hormone antagonists, hycamtin, hydroxy urea, idarubicin, ifosfamid, imatinib, irinotecan, letrozol, leuprorelin, lomustin, melphalan, mercaptopurine, methotrexate, miltefosin, mitomycine, mitosis inhibitors, mitoxantron, nimustine, oxaliplatin, paclitaxel, pentostatin, procarbacin, tamoxifen, temozolomid, teniposid, testolacton, thiotepa, thioguanine, topoisomerase inhibitors, topotecan, treosulfan, tretinoin, triptorelin, trofosfamide, vinblastine, vincristine, vindesine, vinorelbine, antibiotics with cytotoxic activities. All the present and future cytostatics, or other medicaments including gene therapy could be applied.

Moreover the inventive catheter could also be used for delivering drugs such as anti-inflammatory or cytostatic, anti-proliferative and/or cytotoxic drugs to the treated area.

When the inventive radiofrequency hyperthermia device is used for treatment of inflammatory conditions the radiofrequency hyperthermia device of the present invention can be used in combination with an anti-inflammatory drug treatment such as a non-steroidal anti-inflammatory drug (NSAID), for example, alcofenac, aceclofenac, sulindac, tolmetin, etodolac, fenopren, thiaprofenic acid, meclofenamic acid, meloxicam, tenoxicam, lornoxicam, nabumetone, acetaminophen, phenacetin, ethenzamide, sulpyrine, mefanamic acid, flufenamic acid, diclofenac sodium, loxoprofen sodium, phenylbutazone, indomethacin, ibuprofen, ketoprofen, naproxen, oxaprozin, flurbiprofen, fenbufen, pranoprofen, floctafenine, piroxicam, epirizole, tiaramide hydrochloride, zaltoprofen, gabexate mesilate, camostat mesilate, ulinastatin, colchicine, probenecid, sulfinpyrazone, benzbromarone, allopurinol, salicylic acid, atropine, scopolamine, levorphanol, ketorolac, tebufelone, tenidap, clofezone, oxyphenbutazone, prexazone, apazone, benzydamine, bucolome, cinchopen, clonixin, ditrazol, epirizole, fenoprofen, floctafeninl, glaphenine, indoprofen, niflumic acid and suprofen, or with a steroidal anti-inflammatory drugs, for example, dexamethasone, hexestrol, methimazole, betamethasone, triamcinolone, fluocinonide, prednisolone, methylprednisolone, hydrocortisone, fluorometholone, beclomethasone dipropionate, estriol, clobetasol, diflorasone diacetate, halbetosal propionate, amicinonide, desoximetasone, halcinonide, mometasone furoate, fluticasone propionate, flurandrenolide, clocortalone, predincarbate, aclometasone dipropionate and desonide.

The advantages of the inventive radiofrequency hyperthermia device are that this device and especially the catheter of said device are easily, safely and hygienically applicable and that the catheter is fixed in its position during the treatment time which is preferably 2 - 3 hours. Moreover, the heat generation can be continuously controlled and adjusted and can be regioselectively applied. Furthermore, the catheter is safe enough to be deflated and removed from the lumen or body-cavity, so that the SFC (single-fault-conditions) and the requirements of CE MDD (CE medical device directive) are fulfilled such as appropriate mechanical properties till 80°C.

Advantageous is also that the full system is made from smallest available SMD parts.

### Description of the figures

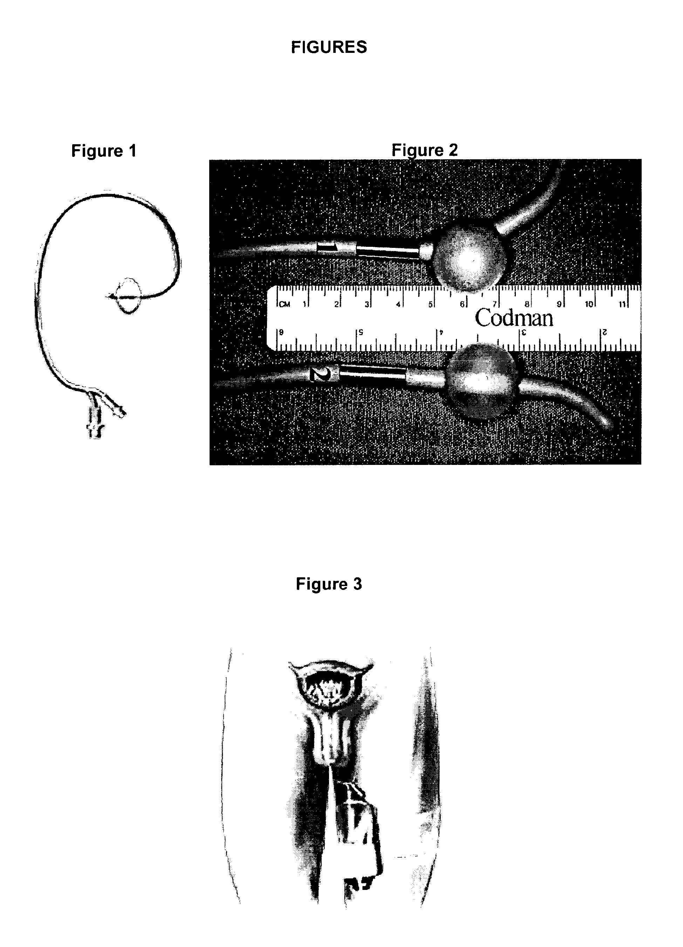

- Figure 1: shows an example of a common Foley catheter.
- Figure 2: shows two embodiments of an oncothermia catheter of the present invention. For transurethral prostate treatment the charriere (Ch) or French gauge (Fg) units in sizes 6-28 Ch16 (5.3mm) Foley catheter is preferable. The Foley-balloon fixes the catheter in the urethra. The Charriere is the outer circumference of the catheter in millimeters. 8-10 Ch is generally used for paediatrics and 12-30 Ch for adult catheters. The most common sizes of retention balloon are 5-10ml and 30ml. The standard length of a male catheter is approximately 45cm and of female catheters is 21cm approximately. It is evident from Fig. 2 that the diameter of the catheter tube is Ch 16 (5.3 mm outside) and that the balloon in its dilated state has a diameter depends on the prostate size, ranges around 2 cm (preferable average 1.8 cm). The balloon is located at the end of the catheter tube just below the guiding wire or guiding head of the catheter and the electrode is like a metal cylinder positioned below the balloon. In the embodiments of the inventive catheter according to Fig. 2 the electrode has a length of about 2 cm. However diameter and length of the catheter tube, the electrode and the balloon as well as the position of the electrode and the balloon can be adjusted to the body lumen or body cavity which should be treated.

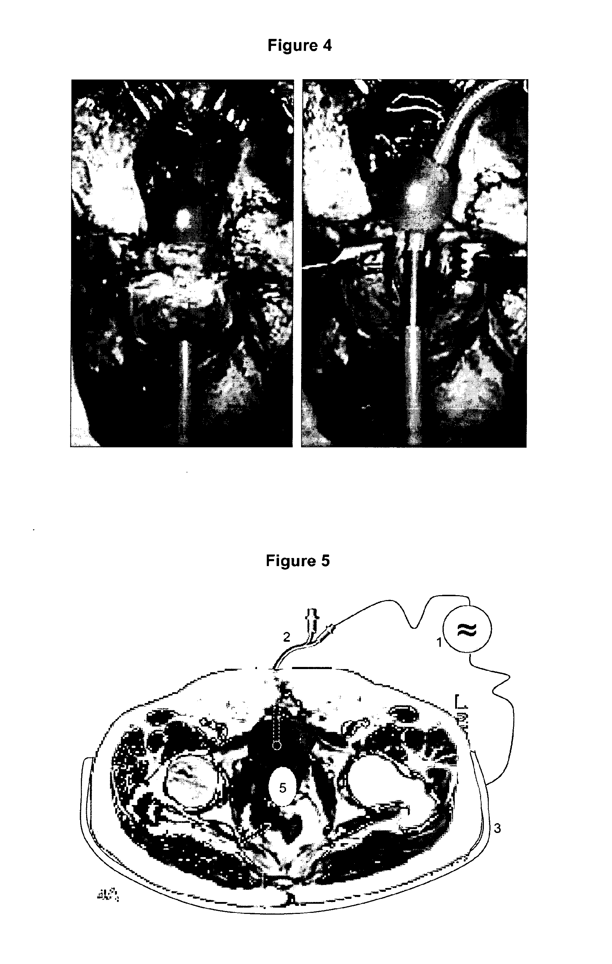

- Figure 3: shows the application of the inventive catheter for prostate treatment The extracorporeal part of the catheter is fixed to the thigh and the catheter tube is inserted through the penis to the prostate where the balloon is inflated to fix the catheter tube and the electrode during the treatment procedure. Fig. 4 shows the catheter tube positioned in the prostate
- Figure 4: shows the inventive catheter inserted into the prostate, (autopsy picture). The left picture shows the inventive catheter which was inserted through the penis into the prostate. The balloon is in the inflated state in order to fix or stabilize the position of the catheter tube in the urethra and thereby the position of the electrode within the prostate. The electrode is not visible since it is complete positioned within the prostate. The right picture shows the identical catheter as in the left picture, while the prostate was cut and pulled apart in order to show the position of the cylindrical electrode which is positioned within the prostate. For the transurethral prostate treatment as shown in Fig. 4 the Ch16 (5.3 mm) Foley-catheter was used.
- Figure 5: shows a schematic arrangement (based on MRI pattern) of the radiofrequency hyperthermia device for prostate treatment. No. 1 is the RF source which is conductively connected to the counter-electrode No. 3 which is extracorporeal and directly attached to patient's skin. The RF source has also a conductive connection through the catheter tube to the electrode position at the end of the catheter which is inserted through the penis into the prostate. In the example of Fig. 5 a Foley-Catheter (No. 2) having a 5.3 mm diameter was used for the prostate treatment. No. 5 is the catheter balloon in the dilated state in order to fix the inserted catheter in its position. A radiofrequency of 339 kHz is applied between the electrodes. The heated area is the area No. 4 between the electrode and the counter-electrode No. 3 which is positioned like a half belt around patient's back.
- Figure 6: Fig. 6B shows the arrangement of the inventive radiofrequency hyperthermia device with the RF source, the catheter inserted through the penis into the prostate and the counter-electrode positioned extracorporeal like a half belt around patient's back. Fig. 6A shows the treated or heated area which is like a triangle between the electrode located in the prostate and the counter-electrode around patient's back. With this arrangement the ventral area of patient's body is not treated and not heated. Thus size and position of the counter-electrode or the relative positions of electrode and counter-electrode are used according to the present invention to regioselectively treat an area of the patient's body. Depending on size and location of a tumor, the inventive radiofrequency hyperthermia device can be used in different arrangements in order to focus the hyperthermia treatment on the malignant tissue.
- Figure 7: Fig. 7B shows the arrangement of the inventive radiofrequency hyperthermia device with the RF source, the catheter inserted through the penis into the prostate and the counter-electrode positioned extracorporeal like a belt (of full belt) around patient's back and abdomen (dorsal and ventral part). Fig. 7A shows the treated or heated area which is approximately oval between the electrode located in the prostate and the counter-electrode positioned completely around the patient and consequently completely around the electrode. With this arrangement the ventral area and the dorsal area of patient's body is equally treated and heated. Thus size and position of the counter-electrode or the relative positions of electrode and counter-electrode are used according to the present invention to regioselectively treat an area of the patient's body. Depending on size and location of a tumor, the inventive radiofrequency hyperthermia device can be used in different arrangements in order to focus the hyperthermia treatment on the malignant tissue.
- Figure 8: shows an embodiment of belt like counter-electrodes. The counter-electrode or the counter-electrodes are incorporated in a belt so that the complete belt (full belt counter-electrode) or the half belt (half belt counter-electrode) or only parts of the belt function as counter-electrodes.
- Figure 9: shows the implemented electronics below the metallic cylinder of the catheter. The RF antenna connection and the two temperature sensor with their isolated filtering circuits is well observable.

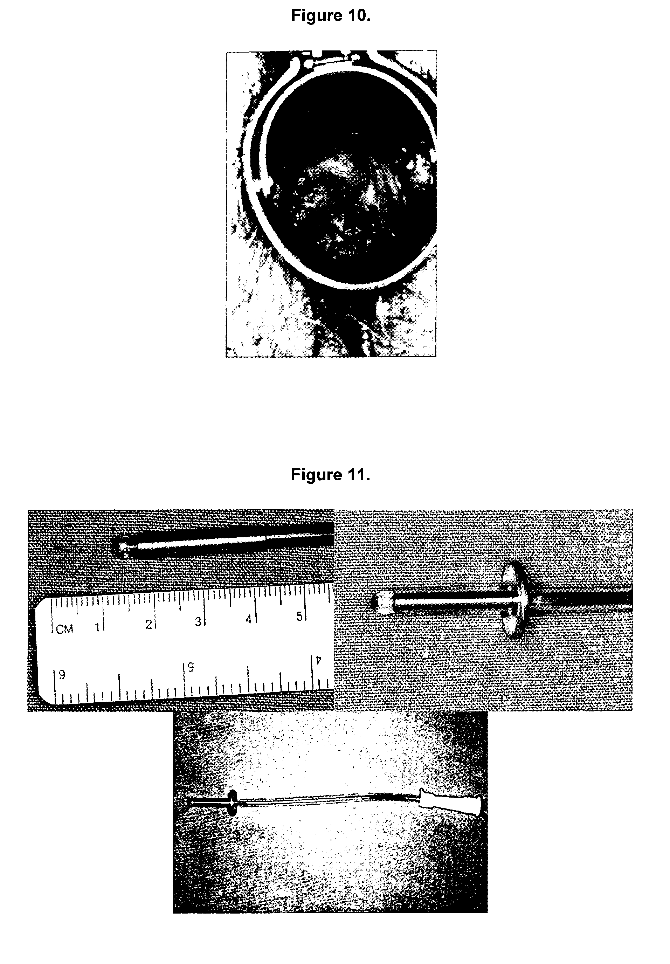

- Figure 10: shows the top of the cervix uteri seeing from the vagina. The cancerous middle is the target of the treatment.
- Figure 11: shows the catheters of the cervix uteri. The shape is developed to put the catheter into the cervix's lumen and cover the cervix top with a annulus neck-piece. This round plate fixes the catheter in the right position and (preferable) treats the top of the cervix (if it is necessary to do so.) The size (without the annulus neck-piece) is shown by a ruler.

### EXAMPLES

### Example 1/a: Prostate cancer treatment

The catheter is inserted to the prostate through the penis and after blowing up the balloon it is fixed on the way that the RF antenna is inside the prostate. (The position could be controlled (preferable) by conventional ultrasound imaging.) Fix the position and connect the urine-container to the catheter. Choose the position which does not heat the closing muscles of the prostate, avoid the after-treatment incontinence. Position the counter electrode in the buttock if the cancer is located on the rear lobe periphery of the prostate. Fix the temperature desired for treatment (preferable it is between 48-52 °C) and start the treatment. The device controls all the parameters automatically. The treatment time is preferable 120 minutes for cancer.

### Example 1/b: Benign prostate hyperplasia (BPH) treatment

The catheter is inserted to the prostate through the penis and after blowing up the balloon it is fixed on the way that the RF antenna is inside the prostate. (The position could be controlled (preferable) by conventional ultrasound imaging.) Fix the position and connect the urine-container to the catheter. Choose the position which does not heat the closing muscles of the prostate, avoid the after-treatment incontinence. Position the counter electrode in the buttock if the cancer is located on the rear lobe periphery of the prostate. Fix the temperature desired for treatment (preferable it is between 48-52 °C) and start the treatment. The device controls all the parameters automatically. The treatment time is preferable 180 minutes for benign prostatic hyperplasia.

### Example 2: Cervical carcinoma

The catheter of the inventive radiofrequency hyperthermia device connected to the RF source is inserted into the end of vagina to the cervix head of a patient suffering from cervical carcinoma (Figure 10). The electrode (Figure 11) which is cylindrically positioned around the catheter tube has a total length of 2.5 cm and a diameter of Ch12. The electrode has annulus neck-piece (1 cm diameter) to fix the position in the cervix. The insertion of the catheter could be followed by neked eye by conventional professional tools to open for seeing the area.

After insertion fix the position of the intracorporeal electrode on the during hyperthermia treatment. The counter-electrode is a flexible woven polymeric material which is coated with a conductive metallic copper layer so that a flexible conductive metallic network is obtained. Such counter-electrode is places on the ventral part of patient's lower abdomen (upper pubic area).

Now between the intracorporeal cylindrical electrode and the extracorporeal flexible counter-electrode a radiofrequency 339 kHz is applied for 60 minutes. Within 20 minutes a temperature of the treated area between the electrode and counter-electrode of 44°C is reached and kept for the remaining treatment time.

The temperature is continuously measured and controlled during treatment through a micro-thermistor located in the catheter guiding head.

The diseased area between the intracorporeal electrode and the counter-electrode was regioselectively heated and the cervical carcinoma was effectively treated by radiofrequency hyperthermia which could be applied in combination with conventional treatments.

### Example 3: Urinary bladder cancer treatment

The catheter of the inventive radiofrequency hyperthermia device connected to the RF source is inserted into the urethra (female) and guided to the urinary bladder for a patient suffering from bladder carcinoma. The electrode, which is cylindrically positioned around the catheter tube has a total length of 2.0 cm and a diameter of Ch12. The bladder has to be filled up by urine-infusion solution mixture electrolyte (the composition is irrelevant, only the fullong op is important for RF current conduction). Follow the electrode by ultrasound, keeping the electrode in the electrolyte at the cancer-vicinity, turning the patient on the right position to make it stable.

The counter electrode also has to be on the opposite side, extra-corporally. The counter-electrode is a flexible woven polymeric material which is coated with a conductive metallic copper layer so that a flexible conductive metallic network is obtained.

Now between the intracorporeal cylindrical electrode and the extracorporeal flexible counter-electrode a radiofrequency 339 kHz is applied for 60 minutes. Do not use ultrasound control together with the RF-treatment. The parallel use could damage both of the devices. However do control the electrode position in every 10-20 minutes with the paused RF-power.

The temperature is continuously measured and controlled during treatment through a micro-thermistor located in the catheter guiding head.

The diseased area between the intracorporeal electrode and the counter-electrode was regioselectively heated and the bladder carcinoma was effectively treated by radiofrequency hyperthermia which could be applied in combination with conventional treatments.

## Claims

1. A catheter for a radiofrequency hyperthermia device useful for the treatment of intraluminar or intracavitary lesions consisting of an RF-electrode, wherein the RF-electrode has no thermal isolation and has a metallic part which is galvanically connected to the tissue and wherein said metallic part allows the precise temperature measurement and the homogeneous temperature distribution and an RF-independent temperature sensor which is isolated from the ground by at least 4kV, wherein the RF-electrode fixed to the catheter is a heat-conductor metallic electrode.

2. A radiofrequency hyperthermia device for the treatment of intraluminar or intracavitary lesions consisting of a catheter with an RF-electrode, wherein, the RF-electrode has no thermal isolation and has a metallic part which is galvanically connected to the tissue and wherein said metallic part allows the precise temperature measurement and the homogeneous temperature distribution and an RF-independent temperature sensor which is isolated from the ground by at least 4kV, and a counter-electrode and a radiofrequency source connected to the RF-electrode and counter-electrode, wherein the RF-electrode fixed to the catheter is a heat-conductor metallic electrode and wherein the counter-electrode is positioned extracorporeal oppositely of the treated area so that the treated area is located between the electrode and the counter-electrode.

3. The radiofrequency device of claim 2 further comprising at least one means for fixing the position of the catheter in the body cavity/lumen, wherein this at least one means is not part of the RF electrode.

4. The radiofrequency device of claim 2, wherein the catheter material is a biocompatible flexible high-temperature tubing.

5. The radiofrequency device of claim 2, wherein the device further comprises a means for temperature measurement and/or a means for treatment control with preprogrammed protocols..

6. The radiofrequency device of claim 2, wherein the RF-electrode is not heated or warmed up itself by the radiofrequency field, and uses conduction heating (not microwave radiation).

7. The radiofrequency device of claim 2, wherein the RF-electrode uses conduction heating and not microwave radiation.

8. The radiofrequency device of claim 2, wherein the counter-electrode is positioned extracorporeal partly or fully around the body, oppositely to the electrode.

9. The radiofrequency device of claim 2, wherein the radiofrequency is 13.56 MHz or any radiofrequency within the range of 10 kHz to 45 MHz preferable obtained by multiplication or division of 13.56 MHz by an integer.

10. The radiofrequency device of claim 2, wherein the electrode is in the form of a cylinder made of pure copper or biocompatible precious metal.

11. The radiofrequency device of claim 10, wherein the copper electrode is coated with a gold-silver alloy.

12. The radiofrequency device of claim 2, wherein the counter-electrode comprises a flexible material coated with a conductive metal. (

13. Use of the radiofrequency device of any one of claims 1 - 12 for the treatment of urethral lesions, malignant or benign prostate tumors, benign prostate hyperplasia, rectum lesions, rectum cancer, colon cancer, anus cancer, rectum cyst, colon cyst, anus cyst, esophagus lesions, esophagus cancer, cervical carcinoma, vagina tumors, vagina cyst, penis cancer, stomach cancer or bladder cancer/cyst.

14. Use of a catheter for the manufacture of a radiofrequency hyperthermia device for the treatment of intraluminar or intracavitary lesions consisting of an RF-electrode, wherein the RF-electrode has no thermal isolation and has a metallic part which is galvanically connected to the tissue and wherein said metallic part allows the precise temperature measurement and the homogeneous temperature distribution and an RF-independent temperature sensor which is isolated from the ground by at least 4kV, wherein the RF-electrode fixed to the catheter is a heat-conductor metallic electrode.

15. Use according to claim 14, wherein the intraluminar or intracavitary lesions are lesions of gastroenterological or gynecological or andrological cavities or lumens.
